# EUROPEAN PATENT SPECIFICATION

(11) **EP 1 603 938 B1**
(45) Date of publication and mention of the grant of the patent: **03.06.2009**
(21) Application number: 03789658.6
(22) Date of filing: 31.12.2003
(51) Int. Cl.: C07K 14/005

(54) **INCLUSION BODIES AS ANTIGENS FOR THE ORAL VACCINATION OF ANIMALS AGAINST CSFV**
EINSCHLUSSKÖRPER ALS ANTIGENE FÜR DIE ORALE IMPFUNG VON TIEREN GEGEN CSFV
CORPS D'INCLUSION UTILISES COMME ANTIGENES DANS LA VACCINATION D'ANIMAUX

(30) Priority: 31.12.2002 PL 35808202
(43) Date of publication of application: 14.12.2005
(73) Proprietor: Instytut Biotechnologii I Antybiotykow, 02-516 Warszawa (PL)
(72) Inventor: PLUCIENNICZAK, Andrzej, PL-04-360 Warszawa (PL); SACZYNSKA, Violetta, PL-02-017 Warszawa (PL); KESIK, Malgorzata, PL-00-137 Warszawa (PL); POREBSKA, Anna, PL-02-615 Warszawa (PL); SZEWCZYK, Boguslaw, PL-80-156 Gdansk (PL); FICINSKA, Jolanta, PL-80-822 Gdansk (PL)
(74) Representative: Witek, Rafal
(86) International application number: PCT/PL2003/000152
(87) International publication number: WO 2004/058806

(56) References cited:
- EP-A- 1 087 014
- WO-A-03/013598
- VAN RIJN P A ET AL: "CSF virus envelope glycoprotein E2 containing one structural antigenic unit protects pigs from lethal CSFV challenge" JOURNAL OF GENERAL VIROLOGY, SOCIETY FOR GENERAL MICROBIOLOGY, READING, GB, vol. 77, 1996, pages 2737-2745, XP002080069 ISSN: 0022-1317
- VAN RIJN P A ET AL: "An experimental marker vaccine and accompanying serological diagnostic test both based on envelope glycoprotein E2 of classical swine fever virus (CSFV)." 5 February 1999 (1999-02-05), VACCINE. 5 FEB 1999, VOL. 17, NR. 5, PAGE(S) 433 - 440 , XP004153825 ISSN: 0264-410X abstract page 434, column 1

## Description

### Field of the invention

The topic of the invention is an oral vaccine and the means and methods used in its production. In general, the invention relates to a method for inducing an immune reaction, encompassing the administration of a vaccine through the gastrointestinal tract. The topics of the invention serve to produce oral vaccines against disease caused by a pestivirus, particularly against Classical Swine Fever.

### Background of the invention

Classical Swine Fever is caused by the Classical Swine Fever Virus (CSFV, also known in the past hog cholera virus). Classical Swine Fever is one of the diseases caused by pestiviruses belonging to the family *Flaviviridae*. Pestiviruses attack animals from the family *Suidae* (swine) and several species of ruminants (cattle, sheep and goats), as well as many species of wild animals. Classical Swine Fever was described for the first time in 1833 in Ohio. Since that time it has dramatically increased its range throughout the world, becoming one of the most limiting economic factors in pig rearing. In 1997 during a Swine Fever epidemic in Spain, over a million animals were destroyed. The losses reached over $140 million US. To date, this dease has not been noted in barely 16 countries. The world organisation, Office International des Epizooties, lists this disease on it's A list, one of the 15 most dangerous animal diseases. Depending on the virulence of the virus, the course of the disease may be: acute, moderate or mild. During an acute attack, the animals exhibit a high fever, alternating constipation and diarrhoea, leukopenia (apoptosis of leukocytes), skin lesions and strong immunosuppression. In a majority of animals, the result of an acute case of Swine Fever is death. A mild case may course asymptomatically. What is more, the disease has a rather long incubation period, from 2 to 14 days, which additionally complicates the control of an epidemic. The most common way of transmitting the disease is contact between sick and healthy animals. Physical transmission is also possible by people, birds and insects, on agricultural implements, feed, vehicles, clothing etc. The infection occurs through the naso-oesophageal pathway. To date, it has proved impossible to treat the disease. Following the pathogen's discover, all herds within a 25 km radius of the epicentre of the epidemic must be eliminated. The virus' resistance is attested to by the fact that it may survive outside of the host organism for as long as a month. Presently, three vaccines exist: one based on the attenuated live C strain of the virus [Terpstra et al. Dtsch Tierarztl Wochenschr 1990; 97 (2): 77-9], one based on a modified infective cDNA clone [Meyers et al. J. Virol., 1996; 70 (3): 1588-95], as well as "marker vaccines" based mainly on the E2 glycoprotein [van Rijn et al., Vaccine 1999; 17 (5): 433-40].

The E2 glycoprotein as well as glycoproteins E0 and E1 comprise the viral envelope of the Swine Fever virus. The pathogen's virulence is most effectively neutralised by antibodies directed against E2 protein epitopes (Weiland et al. 1992. J. Virol. 66:3677). Protein E2 possesses characteristic epitopes at the N-terminus, which are recognised by antibodies. These are the four domains A, B, C and D, exposed to the outside of the membrane, in which the protein is anchored with the C-end (van Rijn et al., 1994. J. Virol. 68:3934).

In contrast to parenteral immunisation, oral vaccines ensure not only systemic immunity, but also mucous membrane immunity, which is particularly important in preventing gastric parasite infection. The growing interest in oral vaccinations is also fuelled by the ease of application, which facilitates administration on a large scale (Richter L., Kipp B.B., 1999. Curr. Top Microbiol. Immunol., 240. 159-176). The first attempts have been undertaken to produce oral vaccines. Description WO00/37610 shows vectors for the manufacture of immunogenic polypeptides, such as HBsAg, in edible plant tissues. Transgenic plants produced in this way are used to manufacture oral vaccinations.

These are not without their faults, however. The difficulty lies in ensuring adequate and precise vaccinating antigen doses. The level of the synthesised antigen varies in different tissues and specimens of the transgenic plants obtained

Descriptions US 5 770 214. US 5 811 105 and US 5 804 194 are related to the application of attenuated strains of *Salmonella typhi* as oral vaccines.

Description US6290962 published 2001.09.18 describes a method for inducing an immune response against *Helicobacter* based on the administration of an immunogenic amount of antigen which was a *Helicobacter* urease or its subunit. Similar oral vaccines containing a recombinant *Helicobacter* urease protein produced in *E.coli* cells are the topics of Polish patents PL 179149 and PL 174448.

A relative lack of experience in oral immunisation and the uncertain fate of the antigen in the gastrointestinal tract cause the conditions of this form of immunisation to be less well defined than for parenteral vaccination. Additionally, oral immunisation carries the risk of the induction of food-based tolerance (McGhee J.R. et al., 1999. w: Mucosal Immunology (Ogra P.L., Lamm M.E., Bienenstock J., Mestecky J., Strober W., McGhee J.R., red), str. 485-505. Academic Press).

Description WO 98/32427 published 1998.07.30 describes a polymer used to prepare coated oral vaccines, with a delayed release of the active substance.

Regardless of the way of administering, the form of the antigen warrants how immunogenic it is.

The acquisition of immunity as a result of vaccination requires the maintenance of the correct immunisation regime, which encompasses factors such as the accurate description of factors such as the size and number, as well as the frequency of individual doses. The acidic pH of gastric juices, proteolytic enzymes and gut peristalsis cause effective doses in oral vaccination to be much higher than in parenteral vaccination. At the same time, however, the administration of an overly high dose of antigen can cause the above mentioned food tolerance. This does not mean that the application of low antigen doses can not induce food tolerance. To date, the basic mechanisms governing this phenomenon have not been discerned, and no standard method has been devised to solve the problem of induced tolerance. It is thought that the phenomenon of tolerance is to some degree dependent on the above mentioned factors, such as the size of the dose, the dosing schedule and the type of antigen (Tacket C.O., Mason H.S., 1999. Microbes and Infection 1. 777-783). The perspectives of producing the appropriate adjuvants, optimal vaccine compositions, efficacious doses and oral vaccination schedules seems to be remote, in light of current research. (Makela P.H., 2000. FEMS Microbiology Rewiews 24. 9-20).

It seems that the prerequisite for immunising through mucous membranes is a multiple administration of the antigen in doses several times higher than the effective doses for parenteral administration. More preferable are antigens forming multimeric groups. The justification of adjuvant usage is also uncertain. In the case of edible plant vaccines this seems to be undesirable, though for some antigens it may be essential (Richter L., Kipp B.B, 1999. Curr. Top Microbiol. Immunol. 240. 159-176).

To summarise, due to the nature of oral vaccinations it seems that it is much more difficult to establish a protocol of effective gastrointestinal immunisation, than it is for immunisation using traditional methods. For each individual medicament this is a separate challenge, connected with a great risk of failure.

There are a number of indicators of the efficacy of immunisation through mucous membranes, including the gastrointestinal tract. These include the level of secreted antigen-specific antibodies of the IgA class (S-IgA) connected with the mucous membranes, the IgG and IgA class antibody titre in the serum, the IgG class antibody profile and the cytokin profile. In order to determine cellular immunity, lymphocyte cytotoxicity tests are used.

The CSFV genome is a single strand of 9.5 to 12.5 kbp and positive polarity. The strand contains one reading frame coding one polyprotein of around 4000 amino-acids. The latter undergoes co- and posttranslational processing into the four structural proteins C, E0. E1. E2 and non-structural proteins, ie. Viral proteases. The virion strucuture is not yet known. It is known that the virion is an icosahedral particle of around 50nm in diameter, and that it is composed of a spherical nucleocapsid and envelope. The nucleocapsid is composed of many molecules of protein C, whereas the envelope is composed of membrane phospholipids and the E0. E1 and E2 surface glycoproteins. The proteins form homo- (E0 and E2) and heterodimers (E1-E2) via disulphide bridges. Only then are they incorporated into the viral envelope (Meyers G., Thiel H.J 1996. van Rijn P. A., van Gennip H. G. P., de Meijer E. J, Moormann R. J. M 1993. Thiel H. J., Stark R., Weiland E., Rumenapf T., Meyers G. 1991). The E1 and E2 proteins possess transmembrane domains, which anchor them in the membrane (van Rijn P. A., van Gennip H. G. P., de Meijer E. J, Moormann R. J. M 1993. van Rijn P. A.,Miedema G. K. W, Wensvoort G, van Gennip H. G. P., Moormann R. J. M. 1994). All of the envelope glycoproteins have immunogenic properties. It is thought that protein E2 is the strongest antigen, stronger than E0 and E1.

Patent description US6207165 (published 2001.03.27) describes a solution relating to a vaccine protecting swine from pathological states caused by diseases of the respiratory and reproductive tract. In this solution, an immunogenic composition is presented which induces an immune response against CSFV, containing a plasmid coding at least one of the E1 or E2 proteins, and which undergoes expression *in vivo* in the host cell.

Patent description US5965134 (published 1999.10.12) describes immunogenic pestivirus polypeptides, particularly of CSFV, and particularly non-structural protein p10 and vaccines and diagnosis using the described polypeptides or nucleic acid molecules.

### THE GOAL OF THE INVENTION

Despite the above described research devoted to the production of a vaccine against CSFV, a need still exists to produce effective tools for the easy manufacture of effective vaccines immunising against this virus.

In the light of the above presented state of technology, the purpose of the present invention is the production of effective and easily obtained specifics which may be applied in oral vaccinations against classical swine fever, which would facilitate the acquisition of systemic and mucous membrane immunity. The treatments according to the present invention should be easy to apply and lacking in other known shortcomings of the preparation and application of vaccines. The latter include relatively high costs of treatment preparation, and a danger of accidental blood poisoning during vaccination. Oral vaccinations according to the present invention should ensure effective immunisation through the gastrointestinal tract.

The next purpose of the present invention is the production of the means which could be used to produce an effective, easy to administer and easily available vaccine caused by a pestivirus, particularly against Classical Swine Fever. A particular goal of the present invention is the production of recombined proteins which would contain selected antigens from the Classic Swine Fever virus, and tools facilitating their expression in *E. coli*. A particular goal of the present invention is the production of the means facilitating the production of recombined proteins which could be used to produce edible vaccines against diseases caused by pestiviruses, particularly against Classical Swine Fever.

Unexpectedly, such a described goal and a solution to problems described in the state of technology have been achieved in the present invention.

### SUMMARY OF THE INVENTION

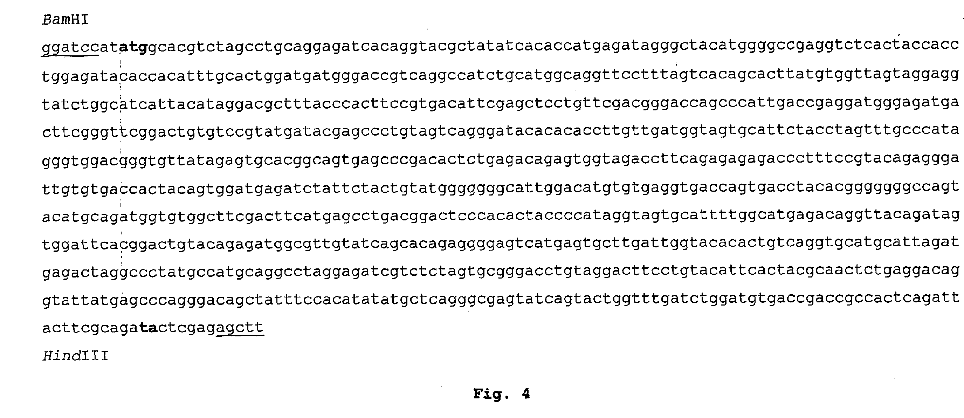

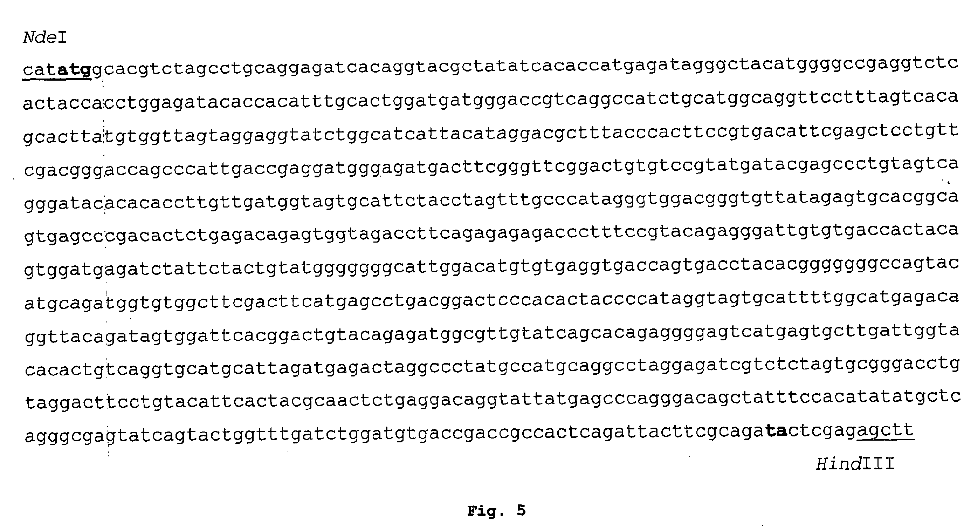

The topic of the present invention is a polypeptide produced in a genetically modified microorganism containing the amino-acid sequence from the E2 protein sequence of the pestivirus, in particular CSFV. Preferentially, it is in the form of inclusion bodies. A preferential embodiment of the present invention is a polypeptide according to the present invention which contains the amino-acid sequence from the E2 protein sequence of the pestivirus, in particular CSFV lacking the transmembrane fragment. Particularly preferentially, the polypeptide according to the present invention contains the sequence coded by the sequence presented in Fig. 4 or Fig. 5.

The topic of the present invention is also the sequence coding a polypeptide coding a polypeptide, which contains a nucleotide sequence coding an aminoacid sequence of the pestivirus E2 protein, particularly of CSFV. Preferentially, the sequence according to the present invention contains the nucleotide sequence coding the amino-acid sequence of the pestivirus E2 protein, particularly of CSFV, lacking the transmembrane fragment, and particularly that which is presented in Fig. 4 or Fig. 5.

The topic of the present invention is also a construct containing the sequence coding a polypeptide, **characterised in that** it is a sequence coding a polypeptide according to the present invention, defined above. Preferentially, the construct according to the present invention is a plasmid produced by the inclusion of the sequence coding the polypeptide into a pIGDM1 plasmid, and particularly preferentially the pIGCmT7 plasmid.

The topic of the present invention jest also a the bacterial cell transformed with the expression construct containing the sequence coding a polypeptide, **characterised in that** the construct contains a sequence according to the present invention defined above as the sequence coding a polypeptide. Preferentially, the the bacterial cell according to the present invention contains a plasmid produced through the inclusion of a sequence coding the polypeptide into the pIGDM1 plasmid, preferentially the pIGCmT7 plasmid. Preferentially, the bacterial cell according to the present invention is a strain of *E.coli*. A specialist is also able to utilise any other given microorganism available for the technology as a strain capable of expression.

The topic of the present invention are also inclusion bodies containing the above defined polypeptide above defined polypeptide according to the present invention.

The topic of the present invention jest also a method for the production of an oral vaccine against disease caused by a pestivirus, particularly against Classical Swine Fever, **characterised in that** it encompasses the transfection of E. *coli* cells with an expression vector, the expression of the polypeptide coded by this vector, the isolation of inclusion bodies containing the produced polypeptide used in the production of a vaccine, where the vector contains a nucleotide sequence coding a polypeptide sequence according to the present invention defined above.

The topic of the present invention is also the application of the polypeptide according to the present invention defined above to produce a vaccine against disease caused by a pestivirus, particularly against Classical Swine Fever.

The topic of the present invention is also a vaccine against disease caused by a pestivirus, particularly against Classical Swine Fever, containing an antigen and possibly a pharmaceutically acceptable carrier and/or adjuvant, **characterised in that**, as the antigen it contains the above defined polypeptide according to the present invention, particularly in the form of inclusion bodies.

The topic of the present invention is also the application of inclusion bodies according to the present invention to produce an oral vaccine against disease caused by a pestivirus, particularly against Classical Swine Fever.

The vaccines are appropriate proteins, whose expression was achieved in *E. coli* bacterial cells of the strain BL21(DE3), transformed with the expression plasmid pIGCmT7 containing a given insert. The proteins were isolated from the *E. coli* bacterial cells in the form of inclusion bodies.

### THE VIRTUES OF THE INVENTION

The present invention possesses many advantages over those known from the solutions known from the state of technology. A very effective method has been devised of inducing the production of antibodies in mucous membranes. Due to the fact that CSFV infections occur through the mucous membranes, the appropriate method of administering the said vaccine is orally, as only this type of vaccine induces a particularly strong immune reaction in the natural site of penetration for the virus. The vaccine produced is easy to administer, and lacks the other known negative aspects of preparation and application of classical vaccines, such as the high cost of production and the danger of accidental blood poisoning during vaccination.

The expression of the antigen occurs in bacterial cells. The homogeneity of the composition produced, and thus precise dosages, are guaranteed by the simple organisation of the selected host and the possibility to control the expression of the vaccinating antigen to be isolated and used in the form of inclusion bodies.

### BRIEF DESCRIPTION OF THE DRAWINGS

The figures included facilitate the clearer explanation of the nature of the present invention.
Figure 1 represents a map of the pIGCmT7 plasmid, where: ARG t-RNA is the gene of arginine tRNA for the codons AGA and AGG, ORI is the origin of replication of the pPIGDM1 plasmid, Cm-R is a chloramphenicol resistance gene, T7 is a promoter, and stop is a transcription terminator;
Figure 2 represents an electrophorogram of the isolated E2 protein of CSFV lacking the transmembrane fragment w in the form of inclusion bodies. Lanes: 1) Protein mass standard LMW 14.4 kDa, 20.1 kDa, 30.0 kDa, 43.0 kDa, 67.0 kDa, 97.0 kDa, 2) isolated E2 protein of CSFV lacking the transmembrane fragment in the form of inclusion bodies.
Figure 3 represents a protein composition administered to mice. Lanes: 1) Protein mass standard LMW 14.4 kDa, 20.1 kDa, 30.0 kDa, 43.0 kDa, 67.0 kDa, 97.0 kDa; 2) and 3) isolated E2 protein of CSFV lacking the transmembrane fragment; 4)-7) albumin samples with the following masses 1µg, 2µg, 4µg, and 6µg of pure protein; 8) isolated E2 protein of CSFV lacking the transmembrane fragment in the form of inclusion bodies.
Figure 4 represents the BresciaE2_Bez_Mem_N sequence coding the E2 protein of CSFV lacking the transmembrane fragment. The fragment is inserted into the pBluescript SK(-) vector into sites of digestion by the restriction nucleases restriction nucleases *Bam*HI and *Hind*III.
Figure 5 represents the BresciaE2_Bez_Mem_N sequence coding the E2 protein of CSFV lacking the transmembrane fragment. The fragment is inserted into the pIGCmT7 vector into sites of digestion by the restriction nucleases restriction nucleases *Nde*I and *Hind*III.
Figure 6 represents the results of the immunogenicity assay of the E2 antigen of CSFV from a transformed strain of *E. coli*. The mice (BALB/c) were twice injected subcutaneously and intramuscularly. The booster dose was applied intravenously. Each time approximately 20 µg of antigen E2 with Freund's complete adjuvant was applied.
Figure 7 represents the results of an assay for the presence of IgG class antibodies in the blood serum of mice fed inclusion bodies containing ∼10 µg of the E2 antigen of CSFV. The antigen was administered on days 0 and 26 of the experiment.
Figure 8 represents the results of an assay for the presence of IgA class antibodies in the faeces of mice fed inclusion bodies containing ∼10 µg of the E2 antigen of CSFV.
Figure 9 represents the results of an assay for the presence of IgG class antibodies in the blood serum of mice fed inclusion bodies containing ∼50 µg of the E2 antigen of CSFV. The antigen was administered on days 0 and 26 of the experiment.
Figure 10 represents the results of an assay for the presence of IgA class antibodies in the faeces of mice fed inclusion bodies containing ∼50 µg of the E2 antigen of CSFV.

Below are example embodiments of the above defined present invention.

### Example 1. The production of an E. coli strain expressing vaccine antigens.

### Plasmids

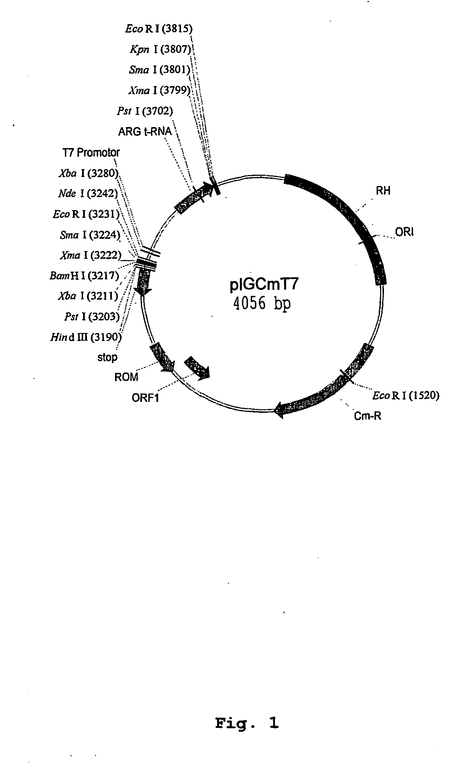

Bacterial expression plasmid pIGCmT7: the pIGCmT7 plasmid (4056 bp.) (Fig.1) was produced in the laboratory of Prof. Andrzej Plucienniczak at the Institute of Biotechnology and Antibiotics in Warsaw, based on the pIGDM1 plasmid (Mikiewicz D. et al., Plasmid 1997), which is a plasmid of the ColE1 group of *Enterobacter agglomerans*. The pIGCmT7 plasmid was produced by the addition of a chloramphenicol resistance gene, a polilinker with the T7 phage RNA polymerase promoter, and a transcription stop codon sequence from the T7 phage.

Non-expression bacterial plasmids: to clone DNA fragments following PCR amplification, the pBluescript SK(-) plasmid from Stratagene was used (the sequence is available from Gene Bank under accession No. X52324 S52394). This plasmid possesses: an origin of replication Col E1. promoters for the T3 and T7 phage RNA plymerases flanking the polylinker, and an ampicilin resistance gene.

### Initial insert sequences

### The sequence of the E2 antigen of the Brescia strain of Swine Fever virus

The nucleotide sequence coding the E2 antigen of the Brescia strain of the Swine Fever virus, 1504 bp long, cloned into the *Pst*I, *Kpn*I restriction sites in the pEVhis13HCVE1 plasmid was obtained from Prof. Szewczyk of Gdask University. The sequence is available from Gene Bank under accession No. M31768.

### Directions for the production of inserts in the vectors.

The coordinates given in the descriptions of production of fragments included into vectors refer to the coordinates in the E2 ntigen sequence deposited in the American Gene Bank.

Directions for the production of the BresciaE2_Bez_Mem_N fragment inserted into the pIGCmT7 expression vector.

From a plasmid containing the full nucleotide sequence of the E2 antigen of CSFV, a fragment 1023 bp long, bp 2428 to 3450. was amplified using PCR. The primers, BRESAMA and UBIBREK2. were designed based on the sequence of the gene coding the E2 protein of CSFV lacking the transmembrane fragment. The forward primer, BRESAMA, introduces a change in the nucleotide sequence of the 5' end of the DNA molecule, exchanging guanine 2430 for thymine. The change introduced into the nucleotide sequence does not cause a change in the amino-acid sequence. Additionally, the BRESAMA primer elongates the 5' end of the DNA molecule, introducing an alanine codon, GCA, and recognition sites for the *Nde*I and *Bam*HI restriction nucleases. The UBIBREK2 primer elongates the 3' end of the DNA molecule, introducing a TA termination codon and recognition sites for the *Hind*III and *Xho*I restriction nucleases. Following PCR, the mixture was separated electrophoretically on a polyacrylamide gel. The amplified DNA fragment, 1058 bp long, was eluted from the polyacrylamide gel, and then digested with the *Bam*HI and *Hind*III restriction nucleases, and deproteinated. The fragment obtained was ligated to the pBluescript SK(-) vector digested with the same restriction nucleases and deproteinated afterwards. The product of this ligation was used to transform *E. coli* cells of the DH5α strain. Following isolation of plasmid DNA from the transformed *E. coli* cells, restriction analysis was used to confirm the presence of the BresciaE2_Bez_Mem_N insert. The accuracy of the sequence of the BresciaE2_Bez_Mem_N fragment included into the pBluescript SK(-) vecotr was confirmed by sequencing.

From the pBluescript SK(-) vecotr containing the cloned insert, a fragment was digested out using the *Nde*I and *Hind*III restriction nucleases. Following the digestion, the mixture was separated on a polyacrylamide gel, and the fragment corresponding to the BresciaE2_Bez_Mem_N insert digested with the *Nde*I and *Hind*III restriction nucleases was excised and eluted out. The fragment of DNA obtained was ligated to the pIGCmT7 expression vector digested with the *Nde*I and *Hind*III restriction nucleases. The ligation product was used to transform *E.* cells of the strain DH5α. Following isolation of plasmid DNA from the transformed *E. coli* cells, restriction analysis was used to confirm the presence of the BresciaE2_Bez_Mem_N insert. The accuracy of the sequence of the BresciaE2_Bez_Mem_N fragment included into the pIGCmT7 vector was confirmed by sequencing.

*E. coli* cells, strain BL21(DE3), were transformed with the plasmid containing the confirmed insert.

### Transformation of the E. coli cells

The method used was transformation of competent *E. coli* bacterial cells, as described by J. Sambrook et al.

### Preparation of inclusion bodies and vaccine antigens.

### Expression and isolation of inclusion bodies

BL21(DE3) strain *E. coli* bacteria bearing the appropriate plasmid were cultured on an LB medium containing chloramphenicol (20µg/ml) at 37°C for 3 hours, and were then diluted with a fresh LB medium (1:100). Following 3 hours of shaking at 37°C, expression was induced by the addition of isopropyl-thio-β-D-galactoside (IPTG, to a final solution of 0.1µg/ml). After 3 hours the bacteria were centrifuged out. The cell sediment was suspended in lysis buffer (0.5 M NaCl, 0.05 M Tris-HCl, pH 7.5, 0.01 M EDTA, 0.005 M 2-Mercaptoethanol, lysozyme 0.35 mg/ml) and incubated for 30 minutes at 20°C. Triton X-100 was added to achieve a 1% concentration. The suspension was sonificated and centrifuged. The sediment of inclusion bodies was suspended twice in PBS buffer containing 1% Triton X-100 and sonificated and centrifuged. The isolated inclusion bodies were washed twice with PBS buffer. The resultant inclusion bodies were suspended in PBS buffer.

### The analysis of purified inclusion bodies

The presence of a pure fraction of inclusion bodies was confirmed by electrophoresis in a 15% polyacrylamide gel (SDS-PAGE). The proteins were visualised with Coomassie blue dye.

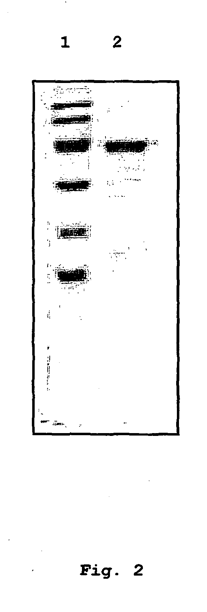

The images of the electrophoresis of isolated inclusion body samples is presented in Figure 2.

### Spectrophotometric determination of protein concentration

Protein concentration was determined by measuring absorbance at λ=595 nm using Bradford reagent.

### Primers and oligonucleotides for PCR.

The primers used to amplify the DNA fragments used were ordered from and manufactured by the Perkin Elmer company.
Name: BRESAMA
5'-GAG GGG ATC CAT ATG GCA CGT CTA GCC TGC AG GA GAT-
Name: UBIBREK2
5'-AA AGC TTC TCG AGT TAT TCT GCG AG TA TCT GAG TG-

Apparatus and other materials:
Enzymes used for cloning: Taq DNA polymerase and buffer (Roche, Expand Long Template PCR System kit, Cat. No. 1 681 834; T4 phage DNA ligase and buffer (USB); T4 polynucleotide kinase and buffer (USB)
Commercially available restriction nucleases were used.
PCR: a PCR PTC-100™ Programmable Thermal Controller thermocycler from MJ Research, Inc.
Sequencing: a Visible Genetics, Inc. sequencer; Amersham sequencing kit, Cat. No. US79840 (according to manufacturer's directions); plasmid DNA purification kit from Kucharczyk Techniki Elektroforetyczne.
Protein concentration determination: a Philips PU 8730 spectrophotometer.
Documentation: Sony Digital Graphics Printer UP 0890 gel documentation system and *Grab it* software.

Bacterial strains: the *E. coli* strains used in the work are:
NM522 *supe thi Δ (lac-proAB) hsd5 F'[proAB*⁺ *lacI^{q} lacZ*Δ*M15]*
DH5α *supE44* Δ*lacU169* (ϕ80 *lacZ*Δ*M15) hsdR17 recA1 endA1 gyrA96 thi-1 relA1*
BL21(DE3) *hsdS gal* (λ*c*Its857 *ind*1 Sam7 *nin*5 *lacUV*5-T7 gene 1)
BZ37 *dam*⁻ *dcm*⁻

The sequences of inserts added to the vectors are presented in Figures 4 and 5.

In the sequence, restriction nuclease recognition sites are underlined, and after digestion with the said enzymes the insert was introduced into the vector. Start and termination codons are shown in bold.

### Example 2. The immunogenicity of the E2 antigen of CSFV from the transformed strain of Escherichia coli

### Purification of the E2 antigen

Inclusion bodies containing the E2 antigen were isolated from the transformed strain of *Escherichia coli* according to the procedure described in Example 1.

The isolated inclusion bodies were separated in a 15% polyacrylamide gel with SDS (SDS-PAGE). A 24 hour transfer of the proteins onto a PVDF membrane (Immobilon TM-P from MILLIPORE) was performed. Next, the fragment of the PVDF membrane bound to the E2 protein of CSFV lacking the transmembrane fragment. The protein was eluted from the membrane in elution buffer (1% Triton X-100. 1% SDS w 50 mM Tris-HCl, pH 9.5), to which 4 volumes of acetone and 1 volume of elution buffer were added. The final volume was left in-20°C. After 12 h the mixture was centrifuged and the resulting precipitate was dried. During the last stage the protein was suspended in physiological saline.

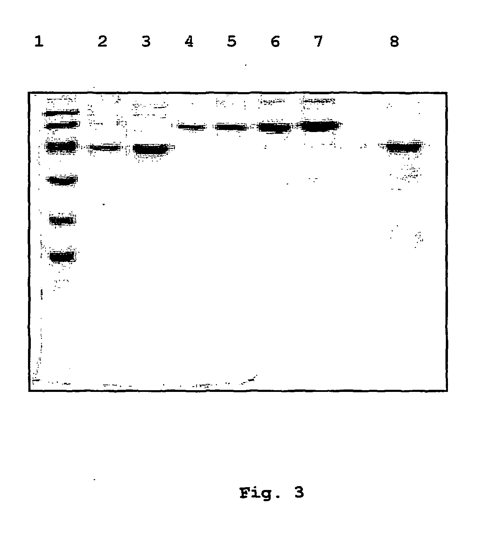

The purity and concentration of the protein preparation administered via injection to mice was determined by polyacrylamide gel electrophoresis in relation to an albumin solution of known concentration (Fig.3 Lanes 2-7).

### Immunisation of mice

The experiment was performed on 10-week old females of the inbred strain BALB/c (Laboratory of Genetics and Animal Rearing, Centre of Oncology, Warsaw). Three mice were inoculated subcutaneously and intramuscularly twice with ∼20 µg of the E2 antigen with Freund's complete adjuvant, with an interval of 2. weeks. One month following the second injection, an intravenous booster dose was administered (∼20 µg of E2 antigen + Freund's complete adjuvant).

### Analysis of blood for the presence of antigen-specific antibodies

Blood was taken from caudal vein 7 and 9 days following the booster dose, using heparin (Sigma) as an anticoagulant. The sera obtained through centrifugation were frozen in aliquots at -20°C.

The assay of the sera for the presence of antibodies against antigen E2 was performed using ELISA. The plates were coated with the E2 antigen of the Brescia strain of CSFV lacking the transmembrane fragment (TM-), obtained in a baculovirus system (Laboratory of Molecular Virology, Gdask University). MaxiSorp plates (Nunc) were coated with the antigen at a concentration of 3µg/ml. The sera were tested at a 1:10 dilution. A negative control was carried out using the sera of un-immunised mice at a 1:10 dilution. A positive control was carried out using the supernatant of hybridoma cultures producing monoclonal antibodies (Mab) against E2 (Laboratory of Bioengineering, Institute of Biotechnology and Antibiotics, Warsaw). The Mab against antigen E2 was produced using the baculovirus product (Laboratory of Molecular Virology, Gdask University).

The plate was developed using antibodies against mouse IgG, specific for the γ chain and tagged with HRP (Sigma), at a dilution of 1:1000. TMB (Sigma) was used as the HRP substrate. The reaction was stopped with 0.5 M H₂SO₄. The absorbance of the samples was measured at 450 nm.

### Results:

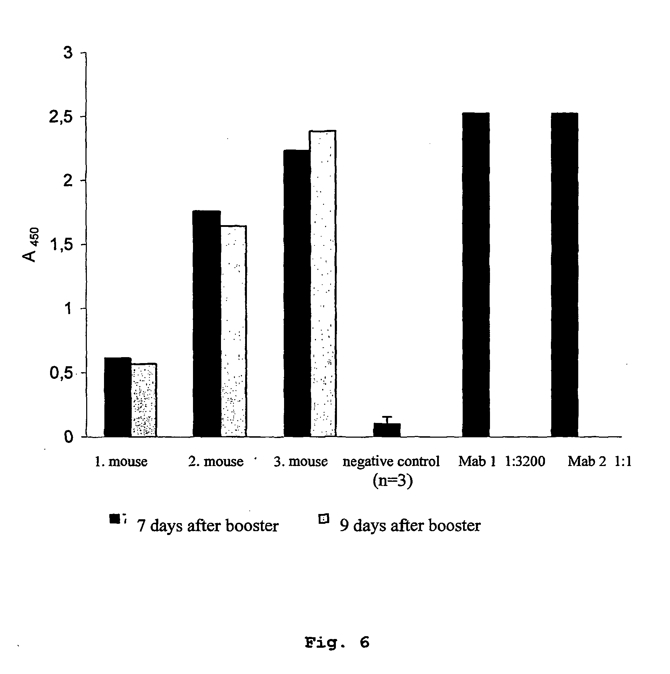

The E2 antigen of CSFV isolated from the transformed strain of *E. coli* and administered parenterally induced an immune response in all mice, as evidence by the presence of antigen specific IgG antibodies in the blood serum (Fig. 6). The results obtained from the application of a standard vaccination procedure demonstrate that:
- the E2 antigen produced by overexpression in *E.coli,* lacking the transmembrane fragment is an active immunogen, like the soluble (TM-) E2 antigen produced in the baculovirus system (Ficińska J. et al. 1999),
- glycosylation is not a prerequisite for this immunogen (recombined proteins produced in bacterial systems are not glycosylated).

The antibodies present in the serum of mice immunised parenterally with the E2 antigen isolated from the transformed strain of *E. coli* react in an ELISA assay with the baculovirus product, just like monoclonal antibodies produced using the baculovirus product (Fig. 6). The indicated reactivity of the antibodies suggests that they are active against the native E2 antigen of CSFV.

The experiment performed proved that the recombined E2 antigen produced through overexpression in *E. coli* is a worthwhile immunogen, and may be applied as a vaccine antigen.

### Example 3. The effectiveness of oral immunisation using ∼10 µg of the E2 antigen of CSFV contained in inclusion bodies

### Production of inclusion bodies

Inclusion bodies containing the E2 antigen were isolated from the transformed strain of *E. coli* according to the procedure described in Example 1.

### Immunisation of mice

A group of mice (three 10-week old females) of the inbred strain BALB/c (Laboratory of Genetics and Animal Rearing, Centre of Oncology, Warsaw) was immunised by the administration of a suspension of inclusion bodies containing 20 µg of protein along with their feed, of which ∼10 µg was composed of the E2 antigen. Two doses were used, with a 26 day interval. The control group was composed of three unimmunised mice.

### Detection of antigen-specific antibodies

The research material was blood and faeces collected in parallel from immunised and control mice. The blood was collected from the caudal vein, 2 days prior to the immunisation, 14 days after the first, and 10, 20 and 30 days following the second feeding with the inclusion bodies. The serum was produced by the centrifugation of the haematocrit using heparin (Sigma) as an anticoagulant. Until analysis, the samples were stored frozen in portions at -20°C.

Faeces were collected every 3-6 days throughout the one month following the second dose of antigen. To suspend the faeces a buffer containing protease inhibitors was used (AEBSF, aprotinin, leupeptin, bestatin). The amount of buffer used was proportional to the amount of faeces collected. Following centrifugation, the supernatant was frozen in portions at -20°C.

The serum and faeces samples were assayed for the presence of antibodies against E2 using ELISA, coating the plate with the E2 antigen of the Brescia strain of CSFV lacking the transmembrane fragment obtained from the baculovirus system (Laboratory of Molecular Virology, Gdask University). Sera of immunised and control mice, diluted 1:10 were tested on MaxiSorp plates (Nunc) coated with the antigen at a concentration of 3 µg/ml. The positive control was the serum of mice immunised parenterally using the E2 antigen isolated from inclusion bodies (Example 2) with the lowest level of antibodies (mouse 1, Fig. 6). To detect antibodies bound to the antigen, antibodies against mouse IgG class antibodies were used, which were specific for the γ chain and tagged with HRP (Sigma), at a dilution of 1:500. The samples prepared from the faeces of immunised and control mice were tested at 1:1 dilution on plates coated with the E2 antigen at a concentration of 5 µg/ml. The plate was developed using antibodies against mouse IgA class antibodies (specific for the α chain) conjugated with HRP (Sigma). The secondary antibody was diluted to 1:500. Both ELISA tests used TMB as a substrate for the peroxidase. The reaction was stopped by the addition of 0.5 M solution of H₂SO₄, and the absorbance was measured at λ=450 nm.

### Results

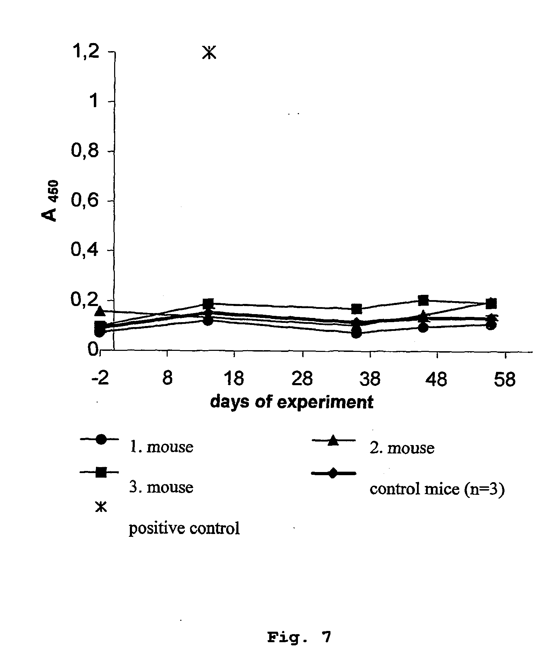

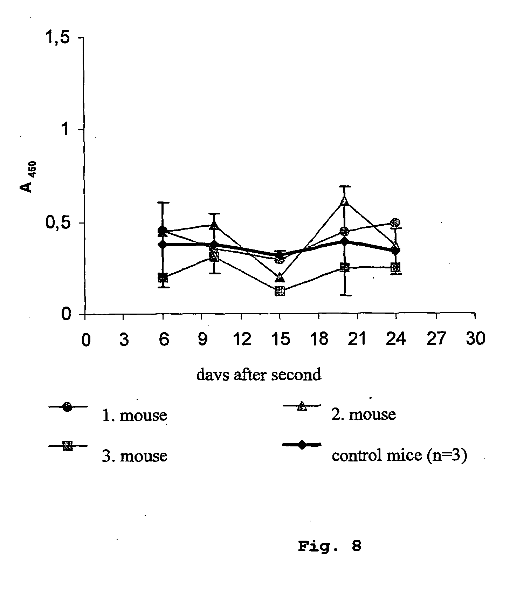

The sera of immunised mice, obtained from blood collected 14 days following the first and 10, 20 and 30 days following the second oral administration of ∼10 µg of the E2 antigen contained in inclusion bodies, do not exhibit a specific reaction to the antigen in the ELISA assay for the presence of IgG class antibodies (Fig. 7). In the faeces of mice fed inclusion bodies, the presence of antibodies was not noted either, in this case of the IgA class of antibody against antigen E2 (Fig. 8). Thus in the experiment performed, a systemic immune system response to the antigen adminsitered was not noted, and neither was a mucous membrane immune response.

The conditions of effective oral immunisation are not as strictly defined as they are for parenteral immunisation, where the use of a standard procedure facilitates the induction of an immune response as in the experiment described in Example 2. Both in the paerenteral immunisation and the oral one, the antigen from the transformed strain of *E. coli* was used. Thus, the cause of the failure was not the inability of the recombined antigen to induce an immune response, but the application of an ineffective immunisation regime.

### Example 4. The effectiveness of oral immunisation using ∼50 µg of the E2 antigen of CSFV contained in inclusion bodies

### Production of inclusion bodies

Inclusion bodies containing the E2 antigen were isolated from the transformed strain of *E. coli* according to the procedure described in Example 3, according to the isolation procedure.

### Immunisation of mice

The experiments wre performed on 10-week old females of the inbred strain BALB/c (Laboratory of Genetics and Animal Rearing, Centre of Oncology, Warsaw). Three mice were immunised twice with a 26-day interval, each time administering an inclusion body suspension with the food, containing 100 µg of protein, of which ∼ 50 µg was comprised of the E2 antigen. Three unimmunised mice were the control group.

### Detection of antigen-specific antibodies

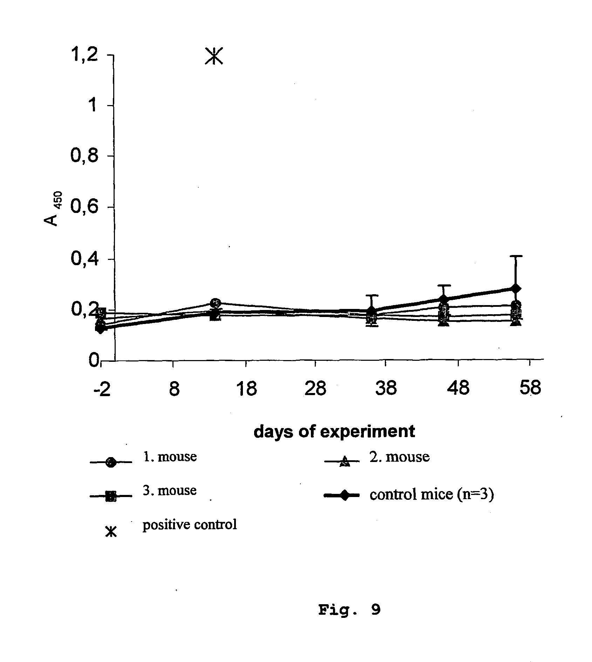

The blood was collected from the caudal vein, 2 days prior to the immunisation, 14 days after the first, and 10, 20 and 30 days following the second feeding with the inclusion bodies containing the E2 antigen. Faeces were collected every 3-6 days throughout the one month following the second dose of antigen. The samples were prepared for testing and tested for the presence of antigen-specific antibodies according to the protocol described in Example 3. Statistical analysis of the data was performed using the Mann-Whitney U-test. In the sera of mice fed inclusion bodies containing ∼50 µg of the E2 antigen of CSFV, antigen-specific IgG antibodies were not detected (Fig. 9). The reactivity of serum samples from blood collected 14 days following the first immunisation, as well as 10, 20 and 30 days after the second administration of the antigen was no different from samples collected 2 days prior to the immunisation and from the serum of control mice.

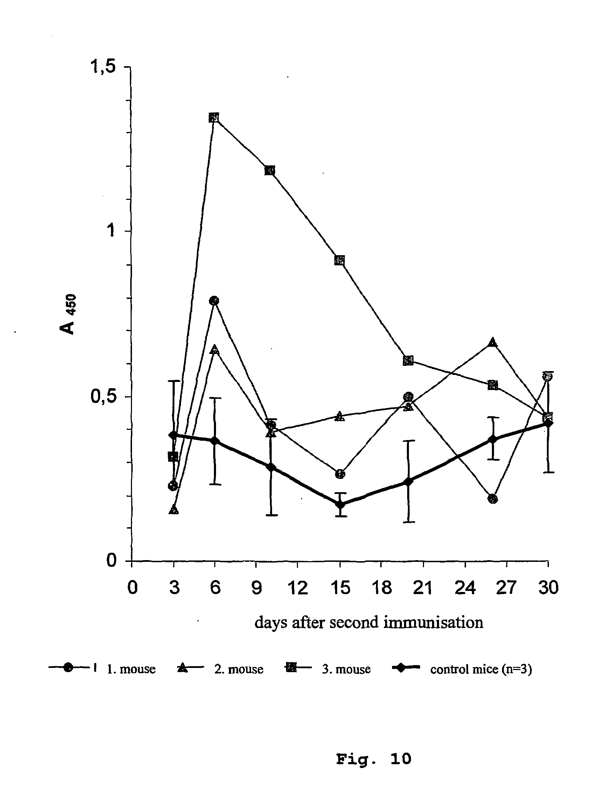

The reactivity of samples from the faeces of immunised mice, which wre collected from 6 to 20 days following the second dose of antigen was significantly (p < 0.001 ) higher than in extracts from the faeces of control mice (Fig. 10). Likewise, statistical analysis of the data obtained from every immunised mouse showed a significant increase in the reactivity of samples taken from the faeces of immunised mice 1. 2. 3 (Fig. 10) in relation to the three control mice (mouse 1. p = 0.025; mouse 2. p < 0.01; mouse 3. p = 0.001). The maximum reactivity of the samples, observed in faeces samples collected 6 days following the II dose was individually 1.8, 2.2 and 3.7 times higher than the average reactivity observed in the control group. Thus the ELISA assay demonstrated the presence of IgA-class antibodies against the E2 antigen of CSFV in the faeces of all immunised animals. The ability to detect these antibodies using plates coated with the E2 antigen produced in the baculovirus system suggests the reactivity of the produced antibodies with a native CSFV antigen.

The results obtained prove that IgA-class antibodies were produced in the gastrointestinal tracts of immunised mice, subsequently detectable in their faeces. The production of these antibodies had a characteristic pattern, similar in all immunised animals (Fig. 10). The first produced antibodies appeared in the faeces of immunised animals between the 3^{rd} and 6^{th} day following the II dose of antigen. Following attainment of a maximum, the level decreased such that by the 30 day following the immunisation they were undetectable by ELISA.

The experimental results indicate that the application of two 50 µg doses of the antigen in inclusion bodies induces a response in the immune system of the mucous membranes of the gastrointestinal tract, evidenced by the production of secreted IgA (S-IgA). The lack of detectable levels of IgG-class antibodies in the serum of the mice is not synonymous with a lack of stimulation of the systemic immune response by an orally administered antigen. As is shown by parenteral immunisation , in many cases this sytem is sensitized effectively, but the production of antibodies is undetectable (Harlow E., Lane D. 1988). In contrast to antibodies circulation in the systemic immune system, an important role is played in immunity to mucous-membrane transmitted diseases, including Swine Fever, by antibodies produced by plasmatic cells of the mucous membranes. This has been shown by such experiments as the oral immunisation with antigens of *Helicobacter pylori* (Description of the invention WO 95/22987. published 31.08.95), and various other bacterial antigens (McGhee J.R., Kyono, H. 1993). Thus, in the experiment performed, the chief goal of oral immunisation has been achieved, unattainable through parenteral immunisation, meaning the stimlation of the immune system connected with the mucous membranes which could protect the latter against infection. Despite a lack of discernible systemic response, the obtained effect of immunization with the E2 antigen contained in inclusion bodies seems to be preferential to such experiements as with the edible vaccine against the Norwalk virus, a virus causing acute inflammation of the stomach and intestines in humans (Mason H. S. et al. 1996). Of eleven mice which reacted with antibody production detected in the blood, following administration of potato roots expressing NV capsid proteins (NVCP) only one mouse produced detectable levels of intestinal IgA.

The results obtained demosntrate that the application of inclusion bodies for the immunisation of animals through the gastrointestinal tract does not require the formulation of specifically designed carriers enhancing the absorption of the antigen through epithelial cells and/or protecting the antigen against degradation in the gut, nor of adjuvants (factors often used in experiments on oral vaccination), to cause a response in the mucous membrane immune system. All this points to an attractive application of inclusion bodies containing the E2 antigen as an edible veterinary vaccine against Swine Fever.

### References

Ficińska J., Szymański P., Szewczyk B., Bieńkowska-Szewczyk K. Expression of CSFV E2 glycoprotein gene in baculovirus system. 1999. Medycyna weterynaryjna 55. 677-680
Harlow E., Lane D. 1988. Immunizations [w]: Antibodies. A laboratory manual. Cold Spring Harbor Laboratory, 53-138
Mason H. S., Ball J.M., Shi J.-J., Jiang X., Estes M.K., Arntzen C.J 1996. Expression of Norwalk virus capsid protein in transgenic tobacco and potato and its oral immunogenicity in mice. Proc. Natl. Acad. Sci. USA 93. 5335-5340
McGhee J.R., Kyono, H. 1993. New perspectives in Vaccine Development: Mucosal immunity to infectios. Infect Agents Dis. 2. 55-73

## Claims

1. A polypeptide in the form of inclusion bodies produced in a genetically modified organism containing the amino-acid sequence of the pestivirus E2 protein, preferably of CSFV, lacking the transmembrane fragment.

2. A polypeptide of claim 1 that contains the sequence coded by the sequence of SEQ ID NO: 1 or SEQ ID NO: 2.

3. A bacterial cell transformed with the expression construct containing the sequence coding amino-acid sequence of the pestivirus E2 protein, preferably of CSFV, lacking the transmembrane fragment.

4. A bacterial cell of claim 3 that contains the construct containing the sequence of SEQ ID NO: 1 or SEQ ID NO: 2.

5. A bacterial cell of claim 3 that is a strain of *E.coli*.

6. Purified inclusion bodies containing a polypeptide containing the amino-acid sequence of the pestivirus E2 protein sequence, preferably of CSFV, lacking the transmembrane fragment.

7. Purified inclusion bodies of claim 6 that are produced in *E.coli*.

8. Purified inclusion bodies according to claim 6, **characterised** that the polypeptide contains the sequence coded by the sequence of SEQ ID NO: 1 or SEQ ID NO: 2.

9. A method for the production of an oral vaccine against a disease caused by a pestivirus, preferably Classical Swine Fever, **characterised in that** it encompasses the transformation of E. *coli* cells with an expression vector, the expression of a polypeptide coded by that vector isolation of inclusion bodies containing the produced polypeptide serving to prepare a vaccine, wherein the vector contains the nucleotide sequence coding the amino-acid sequence of the pestivirus E2 protein sequence, preferably of CSFV, lacking the transmembrane fragment.

10. A method according to claim 9, **characterised in that** the mentioned nucleotide sequence is the sequence of SEQ ID NO: 1 or SEQ ID NO: 2.

11. Use of the polypeptide in the form of inclusion bodies according to any of claims 1 to 2 for preparing a vaccine against disease caused by a pestivirus, preferably against Classical Swine Fever.

12. An oral vaccine against disease caused by a pestivirus, preferably against Classical Swine Fever, containing the antigen and possibly a pharmaceutically permitted carrier and/or adjuvant, **characterised in that** as the antigen contains a purified polypeptide containing the amino-acid sequence of the pestivirus E2 protein sequence, preferably of CSFV, lacking the transmembrane fragment.

13. A vaccine according to claim 12, **characterised in that** the polypeptide is produced in a genetically modified prokaryotic host organism.

14. A vaccine according to claim 12, **characterised in that** the polypeptide is in the form of inclusion bodies.

15. A vaccine according to claim 12, **characterised in that** the polypeptide contains the sequence coded by the sequence of SEQ ID NO: 1 or SEQ ID NO: 2.

16. Use of the purified inclusion bodies according to any of claims 6 to 8 for preparing an oral vaccine against disease caused by a pestivirus, preferably against Classical Swine Fever.

## Patentansprüche

1. Ein Polypeptid in Form von Einschlusskörpern, hergestellt in einem genetisch modifizierten Organismus, enthaltend eine Aminosäuresequenz des E2-Proteins des Pestiviruses, insbesondere des CSFV, der die Transmembrandomäne fehlt.

2. Ein Polypeptid gemäß Anspruch 1, welches eine Sequenz, die durch die Sequenz nach SEQ ID NO:1 oder SEQ ID NO:2 kodiert ist, enthält.

3. Eine bakterielle Zelle, transformiert mit einem Expressionskonstrukt, enthaltend eine Sequenz, die kodiert für die Aminosäuresequenz des E2-Proteins des Pestiviruses, insbesondere des CSFV, der die Transmembrandomäne fehlt.

4. Eine bakterielle Zelle gemäß Anspruch 3, die ein Konstrukt, enthaltend eine Sequenz nach SEQ ID NO:1 oder SEQ ID NO:2, enthält.

5. Eine bakterielle Zelle gemäß Anspruch 3, die ein Stamm von *E.coli* ist.

6. Aufgereinigte Einschlusskörper, enthaltend ein Polypeptid, enthaltend eine Aminosäuresequenz der E2-Proteinsequenz des Pestiviruses, insbesondere des CSFV, der die Transmembrandomäne fehlt.

7. Aufgereinigte Einschlusskörper gemäß Anspruch 6, die in *E.coli* hergestellt sind.

8. Aufgereinigte Einschlusskörper gemäß Anspruch 6, **dadurch gekennzeichnet, dass** das Polypeptid eine Sequenz, kodiert durch die Sequenz nach SEQ ID NO:1 oder SEQ ID NO:2, enthält.

9. Ein Verfahren zur Herstellung eines oralen Impfstoffes gegen eine Krankheit, verursacht durch ein Pestivirus, insbesondere klassische Schweinepest, **dadurch gekennzeichnet, dass** es eine Transformation von *E.coli*-Zellen mit einem Expressionsvektor, die Expression des Polypeptids, das durch diesen Vektor kodiert ist, Isolierung der Einschlusskörper, die das hergestellte Polypeptid enthalten, das zur Herstellung des Impfstoffs dient, umfasst, wobei der Vektor die Nukleotidsequenz enthält, die für die Aminosäuresequenz der E2-Proteinsequenz des Pestiviruses, insbesondere des CSFV, der die Transmembrandomäne fehlt, kodiert.

10. Ein Verfahren gemäß Anspruch 9, **dadurch gekennzeichnet, dass** die genannte Nukleotidsequenz eine Sequenz nach SEQ ID NO:1 oder SEQ ID NO:2 ist.

11. Verwendung des Polypeptides in Form von Einschlusskörpern gemäß einem der Ansprüche 1 oder 2 zur Herstellung eines Impfstoffes gegen eine Krankheit, verursacht durch ein Pestivirus, insbesondere gegen klassische Schweinepest.

12. Ein oraler Impfstoff gegen eine Krankheit, verursacht durch ein Pestivirus, insbesondere gegen klassische Schweinepest, enthaltend das Antigen und eventuell einen pharmazeutisch zugelassenen Träger und/oder Hilfsstoff, **dadurch gekennzeichnet, dass** er als Antigen ein aufgereinigtes Polypeptid enthält, enthaltend die Aminosäuresequenz der E2-Proteinsequenz des Pestiviruses, insbesondere des CSFV, der die Transmembrandomäne fehlt.

13. Ein Impfstoff gemäß Anspruch 12, **dadurch gekennzeichnet, dass** das Polypeptid in einem genetisch modifizierten prokaryotischen Wirtsorganismus hergestellt wird.

14. Ein Impfstoff gemäß Anspruch 12, **dadurch gekennzeichnet, dass** das Polypeptid in Form von Einschlusskörpern vorliegt.

15. Ein Impfstoff gemäß Anspruch 12, **dadurch gekennzeichnet, dass** das Polypeptid die Sequenz, kodiert durch die Sequenz nach SEQ ID NO:1 oder SEQ ID NO:2, enthält.

16. Verwendung der aufgereinigten Einschlusskörper gemäß einem der Ansprüche 6 bis 8 zur Herstellung eines oralen Impfstoffs gegen eine Krankheit, verursacht durch ein Pestivirus, insbesondere gegen klassische Schweinepest.

## Revendications

1. Polypeptide sous la forme de corps d'inclusion produit dans un organisme génétiquement modifié contenant la séquence d'acides aminés de la protéine E2 des pestivirus, de préférence du CSFV, ne possédant pas le fragment transmembranaire.

2. Polypeptide selon la revendication 1, qui contient la séquence codée par la séquence de SEQ ID NO : 1 ou SEQ ID NO : 2.

3. Cellule bactérienne transformée avec la construction d'expression contenant la séquence codant pour la séquence d'acides aminés de la protéine E2 des pestivirus, de préférence du CSFV, ne possédant pas le fragment transmembranaire.

4. Cellule bactérienne selon la revendication 3, qui contient la construction contenant la séquence de SEQ ID NO : 1 ou SEQ ID NO : 2.

5. Cellule bactérienne selon la revendication 3, qui est une souche d'*E. coli*.

6. Corps d'inclusion purifiés contenant un polypeptide contenant la séquence d'acides aminés de la séquence de la protéine E2 des pestivirus, de préférence du CSFV, ne possédant pas le fragment transmembranaire.

7. Corps d'inclusion purifiés selon la revendication 6, qui sont produits dans *E. coli*.

8. Corps d'inclusion purifiés selon la revendication 6, **caractérisés en ce que** le polypeptide contient la séquence codée par la séquence de SEQ ID NO : 1 ou SEQ ID NO : 2.

9. Procédé de production d'un vaccin oral contre une maladie provoquée par un pestivirus, de préférence le virus de la fièvre porcine classique, **caractérisé en ce qu'**il comprend la transformation de cellules d'*E. coli* avec un vecteur d'expression, l'expression d'un polypeptide codé par ce vecteur, l'isolement de corps d'inclusion contenant le polypeptide produit servant à préparer un vaccin, le vecteur contenant la séquence nucléotidique codant pour la séquence d'acides aminés de la séquence de la protéine E2 des pestivirus, de préférence du CSFV, ne possédant pas le fragment transmembranaire.

10. Procédé selon la revendication 9, **caractérisé en ce que** la séquence nucléotidique mentionnée est la séquence de SEQ ID NO : 1 ou SEQ ID NO : 2.

11. Utilisation d'un polypeptide sous la forme de corps d'inclusion selon l'une quelconque des revendications 1 à 2, pour la préparation d'un vaccin contre une maladie provoquée par un pestivirus, de préférence contre la fièvre porcine classique.

12. Vaccin oral contre une maladie provoquée par un pestivirus, de préférence contre la fièvre porcine classique, contenant l'antigène et éventuellement un vecteur et/ou un adjuvant pharmaceutiquement acceptables, **caractérisé en ce que** l'antigène contient un polypeptide purifié contenant la séquence d'acides aminés de la séquence de la protéine E2 des pestivirus, de préférence du CSFV, ne possédant pas le fragment transmembranaire.

13. Vaccin selon la revendication 12, **caractérisé en ce que** le polypeptide est produit dans un organisme hôte procaryote génétiquement modifié.

14. Vaccin selon la revendication 12, **caractérisé en ce que** le polypeptide se trouve sous la forme de corps d'inclusion.

15. Vaccin selon la revendication 12, **caractérisé en ce que** le polypeptide contient la séquence codée par la séquence de SEQ ID NO : 1 ou SEQ ID NO : 2.

16. Utilisation des corps d'inclusion purifiés selon l'une quelconque des revendications 6 à 8, pour la préparation d'un vaccin oral contre une maladie provoquée par un pestivirus, de préférence contre la fièvre porcine classique.


### SEQUENCE LISTING

<110> INSTYTUT BIOTECHNOLOGII I ANTYBIOTYKÓW UNIWERSYTET GDAŃSKI
<120> Inclusion bodies as antigens for the oral vaccination of animals
<130> PZ/0061/RW
<140> PL 358082
<141> 2002-12-31
<160> 2
<170> PatentIn version 3.2
<210> 1
<211> 973
<212> DNA
<213> synthetic
<400> 1
   ggatccatat ggcacgtcta gcctgcagga gatcacaggt acgctatatc acaccatgag      60
   atagggctac atggggccga ggtctcacta ccacctggag atacaccaca tttgcactgg     120
   atgatgggac cgtcaggcca tctgcatggc aggttccttt agtcacagca cttatgtggt     180
   tagtaggagg tatctggcat cattacatag gacgctttac ccacttccgt gacattcgag     240
   ctcctgttcg acgggaccag cccattgacc gaggatggga gatgacttcg ggttcggact     300
   gtgtccgtat gatacgagcc ctgtagtcag ggatacacac accttgttga tggtagtgca     360
   ttctacctag tttgcccata gggtggacgg gtgttataga gtgcacggca gtgagcccga     420
   cactctgaga cagagtggta gaccttcaga gagagaccct ttccgtacag agggattgtg     480
   tgaccactac agtggatgag atctattcta ctgtatgggg gggcattgga catgtgtgag     540
   gtgaccagtg acctacacgg gggggccagt acatgcagat ggtgtggctt cgacttcatg     600
   agcctgacgg actcccacac taccccatag gtagtgcatt ttggcatgag acaggttaca     660
   gatagtggat tcacggactg tacagagatg gcgttgtatc agcacagagg ggagtcatga     720
   gtgcttgatt ggtacacact gtcaggtgca tgcattagat gagactaggc cctatgccat     780
   gcaggcctag gagatcgtct ctagtgcggg acctgtagga cttcctgtac attcactacg     840
   caactctgag gacaggtatt atgagcccag ggacagctat ttccacatat atgctcaggg     900
   cgagtatcag tactggtttg atctggatgt gaccgaccgc cactcagatt acttcgcaga     960
   tactcgagag ctt                                                        973
<210> 2
<211> 968
<212> DNA
<213> synthetic
<400> 2
   catatggcac gtctagcctg caggagatca caggtacgct atatcacacc atgagatagg      60
   gctacatggg gccgaggtct cactaccacc tggagataca ccacatttgc actggatgat     120
   gggaccgtca ggccatctgc atggcaggtt cctttagtca cagcacttat gtggttagta     180
   ggaggtatct ggcatcatta cataggacgc tttacccact tccgtgacat tcgagctcct     240
   gttcgacggg accagcccat tgaccgagga tgggagatga cttcgggttc ggactgtgtc     300
   cgtatgatac gagccctgta gtcagggata cacacacctt gttgatggta gtgcattcta     360
   cctagtttgc ccatagggtg gacgggtgtt atagagtgca cggcagtgag cccgacactc     420
   tgagacagag tggtagacct tcagagagag accctttccg tacagaggga ttgtgtgacc     480
   actacagtgg atgagatcta ttctactgta tgggggggca ttggacatgt gtgaggtgac     540
   cagtgaccta cacggggggg ccagtacatg cagatggtgt ggcttcgact tcatgagcct     600
   gacggactcc cacactaccc cataggtagt gcattttggc atgagacagg ttacagatag     660
   tggattcacg gactgtacag agatggcgtt gtatcagcac agaggggagt catgagtgct     720
   tgattggtac acactgtcag gtgcatgcat tagatgagac taggccctat gccatgcagg     780
   cctaggagat cgtctctagt gcgggacctg taggacttcc tgtacattca ctacgcaact     840
   ctgaggacag gtattatgag cccagggaca gctatttcca catatatgct cagggcgagt     900
   atcagtactg gtttgatctg gatgtgaccg accgccactc agattacttc gcagatactc     960
   gagagctt                                                              968